# EUROPEAN PATENT SPECIFICATION

(11) **EP 4 065 131 B1**
(45) Date of publication and mention of the grant of the patent: **18.09.2024**
(21) Application number: 20815922.8
(22) Date of filing: 30.10.2020
(51) Int. Cl.: A61K 31/77, A61K 9/00, A61K 35/00, A61P 1/10, A61K 35/644, A61K 47/10, A61K 47/12

(54) **RECTAL-USE COMPOSITION FOR THE TREATMENT OF CONSTIPATION**
ZUSAMMENSETZUNG ZUR REKTALEN ANWENDUNG FÜR DIE BEHANDLUNG VON VERSTOPFUNG
COMPOSITION À USAGE RECTAL POUR LE TRAITEMENT DE LA CONSTIPATION

(30) Priority: 28.11.2019 IT 201900022371
(43) Date of publication of application: 05.10.2022
(73) Proprietor: CAPRIKA SRL, 41012 Carpi (MO) (IT)
(72) Inventor: SALVATORE, Cristian, 80014 Giugliano in Campania (NA) (IT); DE MEO, Arturo, 80014 Giugliano in Campania (NA) (IT)
(74) Representative: Studio Torta S.p.A.
(86) International application number: PCT/IB2020/060209
(87) International publication number: WO 2021/105792

(56) References cited:
- WO-A1-2012/120027
- WO-A1-98/53802
- WO-A2-2010/143004
- CN-C- 1 026 464
- GB-A- 917 456
- US-A1- 2013 137 741

## Description

### CROSS-REFERENCE TO RELATED APPLICATIONS

This Patent Application claims priority from Italian Patent Application No. 102019000022371 filed on November 28, 2019.

### TECHNICAL FIELD

The present invention concerns a composition as defined in the claims for use in the treatment of constipation comprising a PEG-based water solution for rectal use.

### PRIOR ART

Chronic constipation is a functional disorder of the gastrointestinal tract characterised by infrequent bowel movements in the absence of identifiable abnormalities. This condition is very common in the general population, with a prevalence ranging from 2% to 35%. Women (female-male ratio 2-3:1), the elderly (over the age of 65), non-Europeans and people living in poorer socio-economic conditions have a greater probability of suffering from this disorder. Like other functional diseases of the gut (for example, irritable bowel syndrome and dyspepsia), chronic constipation entails a considerable reduction in patient quality of life.

Chronic constipation can be associated with a further degree of disability if the patients present faecal impaction in the rectum or in the most proximal segments of the colon.

This can lead to overflow or soiling, which further deteriorates the patient's quality of life.

Patients with constipation often tend to self-medicate by changing their diet (increase in fibre intake) and, above all, by using irritating laxatives.

In addition to changes in lifestyle, which are not always fully effective, doctors can prescribe different types of laxatives. Laxatives are agents that stimulate defecation or modify faecal consistency and ease of passage.

There are at least three broad categories of laxatives based on their mechanism of action: laxatives that form a mass (bulk forming agents), osmotic laxatives and stimulants (also called "irritants"). In some countries (for example the United States), a fourth category is available: laxatives that soften the faeces (stool softeners), such as docusate.

In the United States, approximately 400 million dollars are spent in over-the-counter laxatives and every year approximately 5 million medical prescriptions are issued for the treatment of constipation. However, approximately 50% of patients are still dissatisfied with their treatment. Remedies for constipation are generally unsatisfactory because, although they can ensure regular bowel movements, they do not always solve (and can even worsen) the signs and symptoms (for example pain and abdominal bloating, flatulence and tension) responsible for the negative impact on the patient's quality of life.

As has been known for some time in the sector, glycerol-based water solutions are widely used for the treatment of constipation. Although glycerol is effective in solving constipation, it is known to produce an irritating effect.

WO2012/120027 discloses an aqueous enema comprising PEG, preferably PEG 3000-4500 Dalton, for treating constipation.

There is therefore a need for compositions for the treatment of constipation that are more effective and have fewer undesired effects than the current pharmacological treatments.

The inventors of the present invention have unexpectedly found that the use of a combination of two types of polyethylene glycol (PEG), with different molecular weight, in rectal-use compositions produces improvements in terms of both effectiveness against constipation and side effects.

### SUBJECT OF THE INVENTION

The subject of the present invention is a rectal-use composition by enema for use in treatment of constipation, characterized in that it comprises a water solution comprising (a) 20 to 80% by weight of a first polyethylene glycol having a mean molecular weight ranging from 2000 to 6000 Dalton and (b) a second polyethylene glycol having a mean molecular weight ranging from 200 to 600 Dalton; said first polyethylene glycol being in a quantity comprised between two and six times by weight the quantity of said second polyethylene glycol; said second polyethylene glycol being present in said water solution in a quantity comprised between 5 and 15% by weight.

Preferably, said first polyethylene glycol is in a quantity comprised between 30 and 60% by weight.

Preferably, said first polyethylene glycol is in a quantity comprised between three and five times by weight the quantity of said second polyethylene glycol.

Preferably, said first polyethylene glycol has a mean molecular weight of 4000 Dalton.

Preferably, said second polyethylene glycol has a mean molecular weight of 400 Dalton.

Preferably, said water solution comprises 0.2 to 5%, more preferably 0.5 to 2% by weight, of honey.

Preferably, said water solution comprises 0.1 to 5%, more preferably 0.5 to 2% by weight of an antimicrobial preservative.

The mean molecular weight values of the polymer species reported in the present description and in the claims were calculated with the international standard method ASTM D4001-13.

### PREFERRED EMBODIMENT OF THE INVENTION

Some examples are provided below for purely illustrative and non-limiting purposes.

Three water solutions were prepared (1 - 3). The first two water solutions (1, 2) represent the solution of the invention and are distinguished from each other by the presence or absence of honey, while solution 3 represents a comparison solution comprising only the high molecular weight PEG.

Table I shows the compositions by weight of the three water solutions in 100 µl of distilled water.

**Table I**

| Water solutions | 1 | 2 | 3 |
|---|---|---|---|
| PEG 4000 (mg) | 83 | | 103.7 |
| PEG 400 (mg) | 20.7 | | -- |
| Honey (mg) | -- | 2 | -- |
| Preservatives (mg) | 1.6 | | |

In solutions 1 and 2 of Table I the PEG 4000 and the PEG 400 are present in a % by weight of approximately 40.0 and approximately 10.0 respectively.

The two PEG's are marketed by the company BRENNTAG under the commercial name PEG 400 and PEG 4000 FLAKES.

The preservatives are benzyl alcohol, dehydroacetic acid and water.

The honey is marketed by the company ACEF under the commercial name WILDFLOWER HONEY.

The above water solutions were produced by the following procedure:
The PEG 4000 in flakes was poured into a melter and thermostatted to a temperature of 55°C until a clear uniform solution was obtained. For solutions 1 and 2, the liquid PEG 400 was added to the PEG 4000 solution produced as described above. For all three solutions, the stirring system was activated for 30 minutes. For solution 2, the honey was dissolved in water in a dissolution apparatus by applying a temperature of 45°C. The honey solution prepared as described was added to the solution of PEG 4000 and PEG 400 previously prepared, keeping the resulting solution under stirring for 30 minutes. Lastly, the preservative was added and the solution was kept under stirring for a further 30 minutes.

The viscosity of the water solutions 1 and 3 was measured and it was found that the presence of low molecular weight PEG increases the viscosity. The measurement was taken with an S02 type rotor at a value of 50 RPM. Table II shows the viscosity values identified.

**Table II**

| Water solution | 1 | 3 |
|---|---|---|
| Viscosity (mPa x s) | 117.6-118.4* | 72.0** |

| | | |
|---|---|---|
| * with a reliability % of 14.7 ** with a reliability % of 10.8 | | |

The three water solutions reported in Table I were tested to verify their effectiveness in the treatment of constipation. For comparison, the same tests were repeated using 100µl of pure glycerol (Comp.) (99.5% OP (official pharmacopoeia) grade). The glycerol is marketed by the company BRENNTAG under the commercial name VEGETABLE GLYCERIN 99.5% OP.

For a better evaluation of the effects of the above single water solutions, the test was repeated using 100µl of distilled water (Ref.).

The tests used male CD1 mice weighing 25-30 g (Charles River, Italy) . The animals were kept in cages (5-7 animals per cage) in standard light conditions (with a light/dark cycle of 12 hours), temperature (22 ± 1°C) and relative humidity (60% ± 10%), for at least one week prior to the experimental sessions.

Food and water were available *ad libitum.* Groups of 5-7 animals were used for each experiment.

All the experiments were performed as indicated by Decree-Law 116/92 and by the new EU directives to minimize the number of animals used and their suffering.

The water solutions reported in Table I were used. It should be highlighted that the two solutions of the invention (1 and 2) reported in Table I have the same quantity of PEG 4000 and PEG 400 so as to represent a significant comparison. Furthermore, again to obtain a significant comparison, the comparison solution 3 has a quantity of PEG 4000 equal to the sum of the quantities of PEG 4000 and PEG 400 of solutions 1 and 2 of the invention.

The animals in the same cage received, rectally, a micro-enema of one specific composition of the above five 100µl compositions (Ref., Comp., solution 1 and solution 2) . For each individual cage, the number of faeces was measured after 4h and after 24h. At the same time, the weight of the wet faeces and of the dry faeces was measured 4h after administration and 24h after administration.

Table III shows the results of the above tests.

**Table III**

| | Ref. | Comp. | 1 | 2 | 3 |
|---|---|---|---|---|---|
| After 4 h | | | | | |
| Number of faeces per cage | 15.5 | 29.6 | 34 | 31 | 25.4 |
| Weight of wet faeces | 0.52g | 1.09g | 0.99g | 1.02g | 0.73 |
| Weight of dry faeces | 0.65g | 1.00g | 0.92g | 0.84g | 0.65 |

| After 24 h | | | | | |
|---|---|---|---|---|---|
| Number of faeces per cage | 345 | 307.5g | 375.5 | 354.5 | 316 |
| Weight of wet faeces | 7.90g | 7.30g | 7.59g | 7.35g | 7.75 |
| Weight of dry faeces | 7.60g | 6.81g | 6.96g | 8.17g | 7.01 |

From the values reported in Table III it can be clearly seen that the solutions according to the present invention (1 and 2) are comparable to, if not better than, the glycerol. It was found that, unlike the glycerol, solutions 1 and 2 do not cause the pain relative to the typical irritating effect of the glycerol.

Furthermore, a comparison of the data relative to solutions 1 and 2 with the data relative to the comparison solution 3 clearly highlights the presence of a synergic action between the PEG 4000 and the PEG 400 which leads to an increase in the viscosity and, consequently, an increase in the pharmacological action (laxative effect). The comparison between the data relative to solutions 1 and 2 and the data relative to solution 3 is extremely significant. In fact, solution 3, although it comprises a greater quantity of the type of PEG (high mean molecular weight) with pharmacologically higher action, is less effective due to the absence of the low molecular weight PEG.

The presence of the low molecular weight PEG (between 200 and 600 Dalton) is necessary for the effectiveness of the composition.

In fact, the solutions containing only high molecular weight PEG (between 2000 and 6000 Dalton) are not viscous enough, with the result that the patient will be obliged to remain in a horizontal position for an excessively long time, to avoid the product leaking from the rectal ampulla. Furthermore, it has been hypothesized that the solutions with such a low viscosity do not remain in contact with the rectal mucosa for a sufficient time to guarantee therapeutic efficacy.

The inventors of the present invention have had the merit of solving the problem with the addition of low molecular weight PEG and predicting the viscosizing effect produced by the low molecular weight PEG from its humectant properties. In this way, it was possible to produce a useful viscosizing effect and improve the therapeutic efficacy of the solution overall. The presence of the low molecular weight PEG considerably improves patient compliance (and therefore also effectiveness) because it requires the patient to rest in a horizontal position for a considerably shorter time, ensuring that the solution, which is more viscous, does not leak out and remains inside the rectum.

However, it has been experimentally proved that a concentration of low molecular weight PEG higher than that claimed entails a reduction in the laxative activity. Furthermore, an excessive quantity of low molecular weight PEG also reduces the technological performance of the product since the micro-enema is unstable at temperatures below 18°C, thus resulting solid and non-performing.

Furthermore, from the values of Table II it can be seen that the presence of the honey in the solution guarantees an improvement in therapeutic efficacy.

Lastly, the values reported in Table III show that there is no systemic absorption of the solutions with high and low molecular weight PEG (with or without honey). In fact, the solutions of the invention are active in 4 hours, i.e. in acute phase, whereas they are devoid of activity at 24 hours.

## Claims

1. A rectal-use composition by enema for use in the treatment of constipation, **characterized in that** it comprises a water solution comprising (a) 20 to 80% by weight of a first polyethylene glycol having a mean molecular weight ranging from 2000 to 6000 Dalton and (b) a second polyethylene glycol having a mean molecular weight ranging from 200 to 600 Dalton; said first polyethylene glycol being in a quantity ranging from two to six times by weight the quantity of said second polyethylene glycol; said second polyethylene glycol being present in said water solution in a quantity ranging from 5 to 15% by weight.

2. The rectal-use composition for use according to claim 1, **characterized in that** said first polyethylene glycol is in a quantity ranging from 30 to 60% by weight.

3. The rectal-use composition for use according to claim 1 or 2, **characterized in that** said first polyethylene glycol is in a quantity ranging from three to five times by weight the quantity of said second polyethylene glycol.

4. The rectal-use composition for use according to one of the preceding claims, **characterized in that** said first polyethylene glycol has a molecular weight equal to 4000 Dalton.

5. The rectal-use composition for use according to one of the preceding claims, **characterized in that** said second polyethylene glycol has a molecular weight equal to 400 Dalton.

6. The rectal-use composition for use according to one of the preceding claims, **characterized in that** said water solution comprises 0.2 to 5% by weight of honey.

7. The rectal-use composition for use according to one of the preceding claims, **characterized in that** said water solution comprises 0.5 to 2% by weight of honey.

8. The rectal-use composition for use according to one of the preceding claims, **characterized in that** said water solution comprises 0.1 to 5% by weight of an antimicrobial preservative.

9. The rectal-use composition for use according to one of the preceding claims, **characterized in that** said water solution comprises 0.5 to 2% by weight of an antimicrobial preservative.

## Patentansprüche

1. Zusammensetzung zur rektalen Anwendung zur Verwendung in der Behandlung von Verstopfung durch Einlauf, **dadurch gekennzeichnet, dass** sie eine Wasserlösung umfasst, die (a) 20 bis 80 Gew.-% eines ersten Polyethylenglykols mit einem mittleren Molekulargewicht in dem Bereich von 2000 bis 6000 Dalton und (b) eines zweiten Polyethylenglykols mit einem mittleren Molekulargewicht in dem Bereich von 200 bis 600 Dalton umfasst; wobei das erste Polyethylenglykol in einer Menge in dem Bereich von zwei- bis sechsmal so viel Gewichtsmenge wie das zweite Polyethylenglykol vorliegt; wobei das zweite Polyethylenglykol in der Wasserlösung in einer Menge in dem Bereich von 5 bis 15 Gew.-% vorhanden ist.

2. Zusammensetzung zur rektalen Anwendung zur Verwendung nach Anspruch 1, **dadurch gekennzeichnet, dass** das erste Polyethylenglykol in einer Menge in dem Bereich von 30 bis 60 Gew.-% vorliegt.

3. Zusammensetzung zur rektalen Anwendung zur Verwendung nach Anspruch 1 oder 2, **dadurch gekennzeichnet, dass** das erste Polyethylenglykol in einer Menge in dem Bereich von drei- bis fünfmal so viel Gewichtsmenge wie das zweite Polyethylenglykol vorliegt.

4. Zusammensetzung zur rektalen Anwendung zur Verwendung nach einem der vorhergehenden Ansprüche, **dadurch gekennzeichnet, dass** das erste Polyethylenglykol ein Molekulargewicht von gleich 4000 Dalton aufweist.

5. Zusammensetzung zur rektalen Anwendung zur Verwendung nach einem der vorhergehenden Ansprüche, **dadurch gekennzeichnet, dass** das zweite Polyethylenglykol ein Molekulargewicht von gleich 400 Dalton aufweist.

6. Zusammensetzung zur rektalen Anwendung zur Verwendung nach einem der vorhergehenden Ansprüche, **dadurch gekennzeichnet, dass** die Wasserlösung 0,2 bis 5 Gew.-% Honig enthält.

7. Zusammensetzung zur rektalen Anwendung zur Verwendung nach einem der vorhergehenden Ansprüche, **dadurch gekennzeichnet, dass** die Wasserlösung 0,5 bis 2 Gew.-% Honig enthält.

8. Zusammensetzung zur rektalen Anwendung zur Verwendung nach einem der vorhergehenden Ansprüche, **dadurch gekennzeichnet, dass** die Wasserlösung 0,1 bis 5 Gew.-% eines antimikrobiellen Konservierungsmittels enthält.

9. Zusammensetzung zur rektalen Anwendung zur Verwendung nach einem der vorhergehenden Ansprüche, **dadurch gekennzeichnet, dass** die Wasserlösung 0,5 bis 2 Gew.-% eines antimikrobiellen Konservierungsmittels enthält.

## Revendications

1. Composition à usage rectal par lavement pour une utilisation dans le traitement de la constipation, **caractérisée en ce qu'**elle comprend une solution aqueuse comprenant (a) 20 à 80 % en poids d'un premier polyéthylène glycol présentant un poids moléculaire moyen se situant dans une plage de 2 000 à 6 000 Dalton et (b) un deuxième polyéthylène glycol présentant un poids moléculaire moyen se situant dans une plage de 200 à 600 Dalton ; ledit premier polyéthylène glycol étant en une quantité se situant dans une plage de deux à six fois en poids la quantité dudit deuxième polyéthylène glycol ; ledit deuxième polyéthylène glycol étant présent dans ladite solution aqueuse en une quantité se situant dans une plage de 5 à 15 % en poids.

2. Composition à usage rectal pour une utilisation selon la revendication 1, **caractérisée en ce que** ledit premier polyéthylène glycol est en une quantité se situant dans une plage de 30 à 60 % en poids.

3. Composition à usage rectal pour une utilisation selon la revendication 1 ou 2, **caractérisée en ce que** ledit premier polyéthylène glycol est en une quantité se situant dans une plage de trois à cinq fois en poids la quantité dudit deuxième polyéthylène glycol.

4. Composition à usage rectal pour une utilisation selon l'une des revendications précédentes, **caractérisée en ce que** ledit premier polyéthylène glycol présente un poids moléculaire égal à 4 000 Dalton.

5. Composition à usage rectal pour une utilisation selon l'une des revendications précédentes, **caractérisée en ce que** ledit deuxième polyéthylène glycol présente un poids moléculaire égal à 400 Dalton.

6. Composition à usage rectal pour une utilisation selon l'une des revendications précédentes, **caractérisée en ce que** ladite solution aqueuse comprend 0,2 à 5 % en poids de miel.

7. Composition à usage rectal pour une utilisation selon l'une des revendications précédentes, **caractérisée en ce que** ladite solution aqueuse comprend 0,5 à 2 % en poids de miel.

8. Composition à usage rectal pour une utilisation selon l'une des revendications précédentes, **caractérisée en ce que** ladite solution aqueuse comprend 0,1 à 5 % en poids d'un conservateur antimicrobien.

9. Composition à usage rectal pour une utilisation selon l'une des revendications précédentes, **caractérisée en ce que** ladite solution aqueuse comprend 0,5 à 2 % en poids d'un conservateur antimicrobien.
